# FASCICULE DE BREVET EUROPEEN

(11) **EP 4 240 431 B1**
(45) Date de publication et mention de la délivrance du brevet: **31.07.2024**
(21) Numéro de dépôt: 21799303.9
(22) Date de dépôt: 08.11.2021
(51) Int. Cl.: A61L 2/10, A61L 2/00, B08B 9/00

(54) **APPAREIL DE DÉCONTAMINATION D'UN OBJET CREUX DÉFINISSANT UNE CAVITÉ INTERNE, MACHINE DE DISTRIBUTION ET PROCÉDÉ ASSOCIÉS**
VORRICHTUNG ZUR DEKONTAMINATION EINES EINEN INNENRAUM DEFINIERENDEN HOHLEN OBJEKTS UND ZUGEHÖRIGE AUSGABEMASCHINE UND VERFAHREN
DEVICE FOR DECONTAMINATING A HOLLOW OBJECT DEFINING AN INTERNAL CAVITY, AND ASSOCIATED DISPENSING MACHINE AND METHOD

(30) Priorité: 09.11.2020 FR 2011483
(43) Date de publication de la demande: 13.09.2023
(73) Titulaire: Capsum, 13013 Marseille (FR)
(72) Inventeur: PAFUMI, Yan, eric, 13120 GARDANNE (FR); GOUTAYER, Mathieu, 35400 SAINT MALO (FR)
(74) Mandataire: Lavoix
(86) Numéro de dépôt international: PCT/EP2021/080862
(87) Numéro de publication internationale: WO 2022/096699

(56) Documents cités:
- WO-A1-2010/012915
- GB-A- 495 499
- US-A1- 2012 261 590
- US-A1- 2016 288 397

## Description

La présente invention concerne un appareil de décontamination d'un objet creux définissant une cavité interne, comportant :
- une unité de décontamination , l'unité de décontamination comportant un support destiné à recevoir et à positionner l'objet creux, un organe de décontamination destiné à être introduit dans la cavité interne, et un mécanisme de déplacement relatif de l'organe de décontamination par rapport au support propre à déplacer l'organe de décontamination et le support relativement l'un par rapport à l'autre entre une position de repos destinée au positionnement de l'objet creux dans le support et une position de décontamination, dans laquelle l'organe de décontamination est destiné à être inséré dans la cavité interne de l'objet creux reçu dans le support,
- une unité de commande propre à activer l'organe de décontamination dans la position de décontamination.

L'appareil de décontamination est destiné à être placé notamment dans un espace commercial, pour former un dispositif autonome, avantageusement associé à une machine séparée de distribution de produit en vrac dans l'objet creux. Alternativement l'appareil de décontamination est intégré au sein d'une machine de distribution de produit en vrac dans l'objet creux.

Le produit destiné à être distribué dans l'objet creux dans est par exemple un produit alimentaire, un produit de nettoyage (notamment un détergent), ou un produit cosmétique. De préférence, le produit est un produit cosmétique.

Avant l'industrialisation, le vrac et la consigne avaient leurs places dans tous les commerces. Ce mode de distribution a décliné avec le développement des grandes surfaces, le travail des femmes et l'explosion de l'industrie de l'emballage. Depuis les trente glorieuses, l'emballage a considérablement évolué et les couches d'emballages se sont multipliées. Aujourd'hui, pour des raisons économiques et écologiques, des modes de consommation et de distribution plus vertueux sont en train de réapparaitre et de se réinventer.

On observe notamment un retour en magasin de machines de distribution en vrac de compositions. Ces machines sont adaptées à tous types de compositions, liquides ou solides, notamment alimentaires, détergentes, voire même cosmétiques. A titre illustratif, des machines de distribution de vrac liquide en magasin sont proposées par 3J Development (3JD) ou CoZie. On peut encore citer la machine décrite dans la demande de brevet français déposée sous le n° FR2008422.

Le déploiement de ce type de machines est toutefois freiné par des problématiques sanitaires et réglementaires, dans la mesure où ce mode de consommation et de distribution repose sur la réutilisation d'emballages. Cette réutilisation implique de garantir une propreté et une stérilité satisfaisante de ces emballages avant leur remplissage, sous peine de contaminations, voire de mise en danger, des consommateurs.

Ceci oblige généralement les magasins à mettre en oeuvre une logistique pour pallier cette difficulté, qui passe, soit par une décontamination des emballages directement en magasin, mais qui s'avère généralement non réalisable en raison de manque de place et/ou de l'absence de point d'eau en magasin, soit par un échange avec un emballage neuf ou préalablement décontaminé, qui implique nécessairement le recours à une industrie chargée de collecter les emballages usagés en magasin, les décontaminer et les renvoyer en magasin.

Cependant, ces solutions ne donnent pas entière satisfaction. Les magasins n'ont pas toujours d'espaces suffisants pour stocker les emballages usagés rapportés par les consommateurs. Par ailleurs, ces logistiques sont manifestement en inadéquation avec les objectifs recherchés en termes d'économie et d'écologie des machines de distribution en vrac en magasin. Elles sont en outre coûteuses.

Il est donc très difficile, voire impossible, de proposer aujourd'hui en magasin une solution de décontamination rapide, facile d'utilisation, sécure, économique et écologique des emballages vides rapportés par les consommateurs. Ceci limite, voire empêche, le déploiement de ce nouveau mode de consommation en dépit de ses atouts écologiques et économiques.

Il existe déjà des installations industrielles de décontamination, notamment à source lumineuse, adaptés à la production en chaine d'objets présentant une cavité. A titre illustratif, on peut citer les dispositifs de décontamination décrits dans WO2010012915 et EP2816002.

Toutefois, ces dispositifs de décontamination sont conçus pour l'industrie de production de masse et fonctionnent en continu. Ils ne sont donc pas adaptés à une utilisation ponctuelle en magasin, à savoir par un opérateur, voire le consommateur, et ce, notamment, en termes de dimensions, de souplesse d'utilisation et surtout de sécurité. US2016/288397 décrit un appareil de décontamination avec une unité de commande pour activer l'organe de décontamination.

Un but de l'invention est de fournir un appareil de décontamination d'objets creux, qui soit adapté à une manipulation ponctuelle en magasin par un opérateur, voire par le consommateur, notamment en termes de dimensions, de souplesse d'utilisation et de sécurité, afin de concourir au déploiement des machines de distribution en vrac, notamment pour des compositions alimentaires, détergentes ou cosmétiques.

A cet effet, l'invention a pour objet un appareil du type précité, caractérisé par une enceinte de protection définissant un volume intérieur contenant au moins une partie de l'organe de décontamination et le support au moins dans la position de décontamination, l'enceinte définissant un passage d'accès au volume intérieur et au moins une paroi d'obturation du passage d'accès, la paroi d'obturation et le passage d'accès étant mobiles l'un par rapport à l'autre entre une configuration ouverte du passage d'accès que la paroi d'obturation est propre à occuper dans la position de repos de l'organe de décontamination et une configuration fermée du passage d'accès que la paroi d'obturation occupe dans la position de décontamination de l'organe de décontamination..

L'appareil selon l'invention peut comprendre l'une ou plusieurs des caractéristiques suivantes, prise(s) isolément ou suivant toute combinaison techniquement possible :
- le mécanisme de déplacement relatif de l'organe de décontamination par rapport au support est propre à déplacer l'organe de décontamination par rapport au support entre la position de repos et la position de décontamination, le support étant statique, ou est propre à déplacer le support par rapport à l'organe de décontamination entre la position de repos et la position de décontamination, l'organe de décontamination étant statique, ou est propre à déplacer simultanément l'organe de décontamination et le support entre la position de repos et la position de décontamination ;
- la paroi d'obturation est mobile par rapport au passage d'accès entre la configuration ouverte et la configuration fermée, le passage d'accès étant statique, ou le passage d'accès est mobile par rapport à la paroi d'obturation entre la configuration ouverte et la configuration fermée, la paroi d'obturation étant statique, ou le passage d'accès et la paroi d'obturation sont mobiles simultanément entre la configuration ouverte et la configuration fermée ;
- le volume intérieur de l'enceinte contient l'unité de décontamination au moins dans la position de décontamination ;
- le volume intérieur de l'enceinte comprend une région d'accès au support communiquant avec le passage d'accès, et une région technique, avantageusement séparée de la région d'accès par au moins une cloison,
- l'organe de décontamination est totalement contenu dans la région technique dans la position de repos, et est au moins partiellement inséré dans la région d'accès dans la position de décontamination ;
- la région d'accès et le passage d'accès définissent une alcôve ménagée dans l'enceinte ;
- l'alcôve contient le support, au moins dans la position de repos ;
- la paroi d'obturation occupe la configuration ouverte dans la position de repos ;
- le déplacement relatif de l'organe de décontamination par rapport au passage d'accès de la position de repos à la position de décontamination provoque le déplacement relatif de la paroi d'obturation par rapport au support de la configuration ouverte à la configuration fermée,
   ou/et le déplacement relatif de la paroi d'obturation par rapport au passage d'accès de la configuration ouverte à la configuration fermée provoque le déplacement relatif de l'organe de décontamination par rapport au support de la position de repos à la position de décontamination,
   le déplacement relatif de l'organe de décontamination par rapport au support et le déplacement relatif de la paroi d'obturation par rapport au passage d'accès étant de préférence assujettis l'un à l'autre ;
- dans la position de repos, la paroi d'obturation est déplaçable relativement au passage d'accès entre la configuration ouverte et la configuration fermée ;
- la paroi d'obturation est maintenue au repos dans la configuration fermée ;
- l'unité de commande est propre à piloter le déplacement relatif de l'organe de décontamination par rapport au support entre la position de repos et la position de décontamination ou/et est propre à piloter le déplacement relatif de la paroi d'obturation par rapport au passage d'accès de la configuration ouverte à la configuration fermée ;
- il comprend un premier capteur de détection de la mise en place de l'objet creux dans le support et/ou un deuxième capteur de détection de la configuration fermée de la paroi d'obturation, et/ou un troisième capteur de détection de la position de décontamination, l'unité de commande étant propre à activer l'organe de décontamination lorsque le premier capteur détecte que l'objet est mis en place dans le support ou/et lorsque le deuxième capteur détecte que la paroi d'obturation est dans la configuration fermée, ou/et lorsque le troisième capteur détecte que l'organe de décontamination est dans la position de décontamination ;
- la paroi d'obturation est un panneau déplaçable en translation ou en rotation par rapport au passage d'accès ou l'enceinte comprend un tiroir mobile définissant le passage d'accès, le tiroir étant mobile par rapport à la paroi d'obturation ;
- le support comporte une région de guidage ou/et de réception d'au moins une partie de l'objet creux, notamment d'un col de l'objet creux, propre à fixer la position et l'alignement de l'objet creux dans le support, la région de guidage ou/et de réception étant en particulier un manchon complémentaire d'un col de l'objet creux, une surface de guidage par gravité d'un col de l'objet creux ou une empreinte de réception de l'objet creux de forme complémentaire à l'objet creux, propre à fixer la position et l'alignement de l'objet creux dans le support ;
- le support comporte un volet de maintien de l'objet creux dans la région de guidage ou/et de réception, déplaçable, en particulier par pivotement, entre une position de mise en place de l'objet creux dans la région de guidage ou/et de réception et une position de maintien de l'objet creux dans la région de guidage ou/et de réception ;
- l'organe de décontamination comporte une source lumineuse, en particulier une source d'émission de lumière UV, continue ou pulsée ;
- le support est disposé au-dessus de l'organe de décontamination dans la position de repos.

L'invention a également pour objet une machine de distribution de produit en vrac dans un objet creux définissant une cavité interne, la machine comprenant au moins un réservoir de produit, au moins une buse de distribution destinée à être placée en regard de la cavité interne de l'objet creux, et éventuellement un ensemble de mesure de la quantité de produit introduite dans la cavité interne de l'objet creux, la machine comportant en outre un appareil de décontamination tel que défini plus haut.

L'invention a également pour objet un procédé de décontamination d'un objet creux présentant une cavité interne comprenant les étapes suivantes :
- fourniture d'un appareil de décontamination tel que défini plus haut ;
- mise en oeuvre d'une séquence de décontamination comprenant les phases suivantes :
   * l'organe de décontamination étant dans sa position de repos, la paroi d'obturation étant dans sa configuration ouverte, introduction d'un objet creux présentant une cavité interne dans le support ;
   * passage de la paroi d'obturation dans la configuration fermée et passage de l'organe de décontamination dans la position de décontamination, l'organe de décontamination s'insérant dans la cavité interne ;
   * activation de l'organe de décontamination par l'unité de commande ; et, à la fin de la séquence de décontamination :
      - passage de la paroi d'obturation dans la configuration ouverte et passage de l'organe de décontamination dans la position de repos, l'organe de décontamination sortant de la cavité interne ;
      - retrait de l'objet creux hors du support.

L'appareil selon l'invention peut comprendre l'une ou plusieurs des caractéristiques suivantes, prise(s) isolément ou suivant toute combinaison techniquement possible :
- après le retrait de l'objet creux hors du support, l'unité de commande pilote le passage de la paroi d'obturation dans la configuration fermée et/ou le passage de l'organe de déplacement de la position de repos à la position de décontamination, sans activer l'organe de décontamination ;
- il comporte ensuite les étapes suivantes :
   - fourniture d'un nouvel objet creux à décontaminer ;
   - passage de l'organe de décontamination dans sa position de repos, et/ou de la paroi d'obturation dans sa configuration ouverte,
   - mise en oeuvre d'une nouvelle séquence de décontamination comprenant les phases suivantes :
      * introduction du nouvel objet creux présentant une cavité interne dans le support,
      * passage de la paroi d'obturation dans la configuration fermée et passage de l'organe de décontamination dans sa position de décontamination, l'organe de décontamination s'insérant dans la cavité interne du nouvel objet creux ;
      * activation de l'organe de décontamination par l'unité de commande.

L'invention sera mieux comprise à la lecture de la description qui va suivre, donnée uniquement à titre d'exemple, et faite en se référant aux dessins annexés, sur lesquels :
- [Fig. 1] La figure 1 est une vue schématique, prise en coupe partielle, d'un premier appareil de décontamination selon l'invention, dans une configuration ouverte permettant le chargement d'un objet creux ;
- [Fig. 2] La figure 2 est une vue analogue à la figure 1, dans une configuration fermée dans laquelle la décontamination est mise en oeuvre ;
- [Fig. 3] La figure 3 est une vue analogue à la figure 1 d'une variante d'appareil selon l'invention ;
- [Fig. 4] La figure 4 est une vue analogue à la figure 1 d'une autre variante d'appareil selon l'invention ;
- [Fig. 5] La figure 5 est une vue analogue à la figure 2 de l'appareil selon l'invention représenté sur la figure 4;
- [Fig. 6] La figure 6 est une vue illustrant schématiquement le support d'un objet creux applicable à l'appareil selon l'invention représenté sur la figure 4 ;
- [Fig. 7] La figure 7 est une vue analogue à la figure 6, d'une autre variante d'appareil selon l'invention ;
- [Fig.8] La figure 8 est une vue schématique en perspective d'un appareil de décontamination selon l'invention, associé à une machine de distribution de produit, mais séparé de la machine de distribution de produit;
- [Fig. 9] La figure 9 est une vue analogue à la figure 8 d'un appareil de décontamination selon l'invention, intégré au sein d'une machine de distribution de produit.

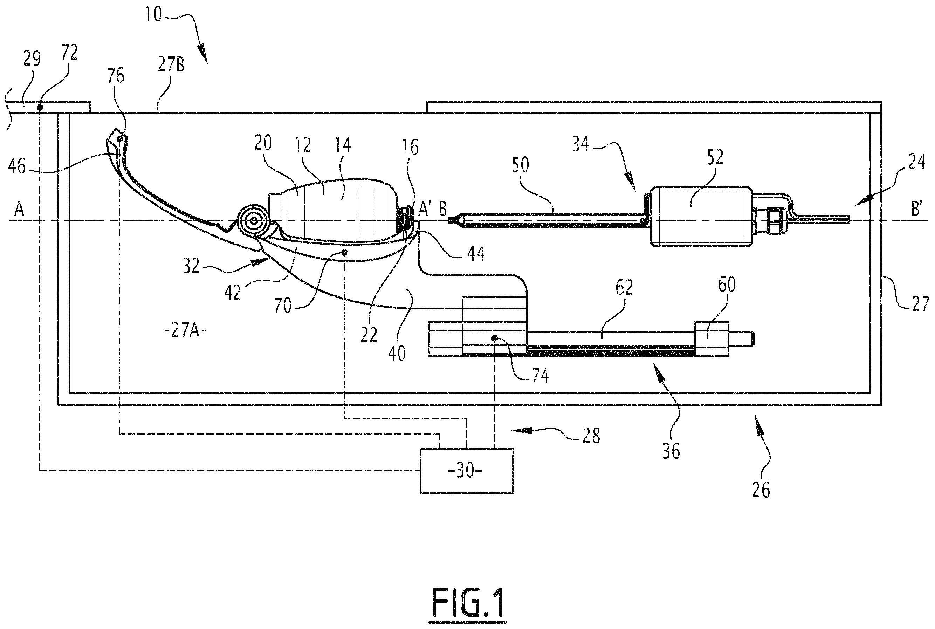

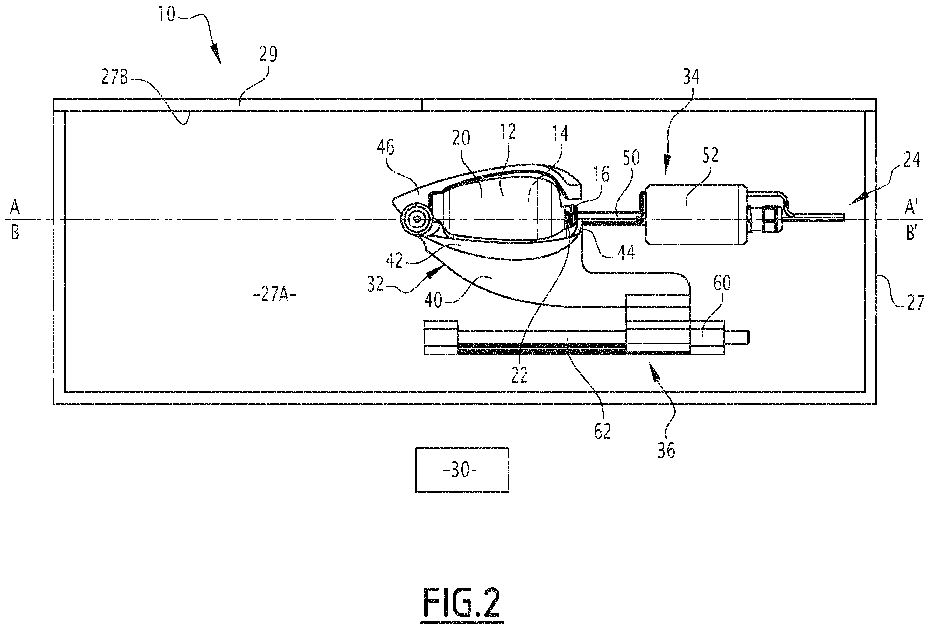

Un premier appareil de décontamination 10 selon l'invention est illustré schématiquement par les figures 1 et 2.

L'appareil 10 est destiné notamment à décontaminer un objet creux 12 comprenant une cavité interne 14 débouchant par une ouverture 16.

Une décontamination de l'objet creux 12 réduit la présence de microorganismes viables, tels que des germes. La décontamination est par exemple une stérilisation de au moins la cavité interne 14 de l'objet creux 12. Une stérilisation est par exemple un traitement permettant de diminuer la charge bactérienne, et notamment d'obtenir une probabilité théorique de présence d'un micro-organisme viable inférieure ou égale à 10⁻⁶ cfu.

La décontamination induit avantageusement une réduction d'au moins 1 log, de préférence d'au moins 2 log, sur un ensemencement d'Aspergillus 1000 UFC/flacon.

L'objet creux 12 est destiné, après décontamination dans l'appareil 10, à recevoir un produit dans la cavité interne 14 à travers l'ouverture 16.

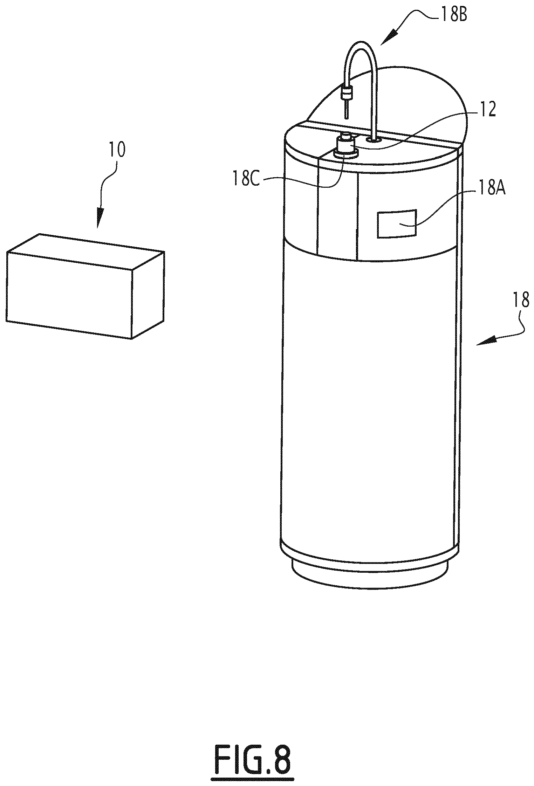

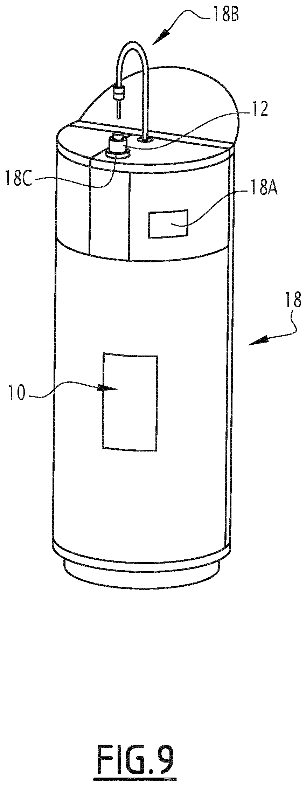

Le produit est délivré par une machine 18 de distribution de produit en vrac, dont des exemples sont illustrés sur les figures 8 et 9. La machine comprend de manière connue un réservoir 18A de produit, une buse de distribution 18B de produit, pour introduire du produit dans l'objet creux 12, et éventuellement un ensemble de mesure 18C de la quantité de produit introduite dans la cavité 14 de l'objet creux 12. L'objet creux 12, après décontamination, est propre à être placé en regard de la buse de distribution 18B pour recevoir une dose de produit.

Dans l'exemple de la figure 8, l'appareil de décontamination 10 est un dispositif autonome avantageusement associé à une machine 18 séparée de distribution de produit en vrac dans l'objet creux 12. Dans l'exemple de la figure 9, l'appareil de décontamination 10 est intégré au sein d'une machine 18 de distribution de produit en vrac dans l'objet creux 12.

La machine est par exemple telle que décrite dans la demande de brevet français n° FR2008422.

Le produit est par exemple solide, pulvérulent ou liquide, de préférence liquide. Il peut être une composition anhydre, aqueuse, ou une émulsion/dispersion simple ou multiple, par exemple telle que décrite dans WO2010063937, WO2018167309, WO2012120043, WO2017046305 ou dans les demandes de brevet français déposées sous les n° FR2005408 et FR2005410.

Avantageusement, le produit est un produit alimentaire, un produit de nettoyage (notamment un détergent), ou un produit cosmétique, de préférence un produit cosmétique.

L'objet creux 12 présentant une cavité 14 est avantageusement un emballage destiné à recevoir le produit.

L'emballage est notamment un packaging cosmétique ou une bouteille.

Cet emballage peut être de toute matière et de toute taille.

Le ou chaque matériau formant l'emballage est adapté à la technologie de décontamination utilisée pour être résistant au vecteur de décontamination utilisé.

Par exemple, un emballage selon l'invention est formé :
- en verre ;
- en plastique, par exemple en polyéthylène (PE), en téréphtalate d'éthylèneglycol (PETG), en polytéréphtalate d'éthylène (PET), en styrène-acrylonitrile (SAN ou ABS), en polypropylène (PP), en chlorure de polyvinyle (PVC) ou en polystyrène (PS) ; ou
- en métal, par exemple en aluminium,
- ou encore en une combinaison des matériaux précités.

La configuration, la taille, ou/et le volume (au moins interne) de l'emballage sont adaptés à la technologie de décontamination/stérilisation utilisée, et dans le cas d'une technologie reposant sur la lumière d'une diode électroluminescente (ci-après DEL) ou sur une lumière ultraviolette (ci-après UV), à la taille de la source lumineuse engendrant la lumière et en particulier à son diamètre.

Par exemple, un emballage destiné selon l'invention présente une contenance comprise entre 5 ml et 1000 ml, de préférence entre 10 ml et 750 ml, en particulier entre 30 ml et 500 ml, et mieux entre 50 ml et 250 ml.

Dans cet exemple, l'objet creux 12 comporte un récipient 20 délimitant la cavité interne 14, et un col 22 définissant l'ouverture d'accès 16 à la cavité interne 14. La cavité interne 14 s'étend de préférence le long d'un axe longitudinal A-A' passant par l'ouverture 16.

En référence aux figures 1 et 2, l'appareil 10 comporte une unité 24 de décontamination, une enceinte 26 de protection, recevant au moins partiellement l'unité de décontamination 24, et avantageusement, un système de capteurs 28. L'appareil 10 comporte en outre une unité de commande 30, propre à piloter au moins l'unité de décontamination 24.

L'enceinte 26 comporte un boitier 27 définissant un volume intérieur 27A destiné à contenir l'unité de décontamination 24 lors d'une séquence de décontamination. Le boitier 27 comporte des cloisons avantageusement opaques et définit au moins un passage 27B d'accès au volume intérieur 27A, destiné à la mise en place de l'objet creux 12 dans l'unité de décontamination 24.

L'enceinte 26 comporte en outre une paroi d'obturation 29, mobile relativement au passage d'accès 27B entre une configuration ouverte du passage d'accès 27B, visible sur la figure 1, autorisant l'introduction de l'objet creux 12 dans l'unité de décontamination 24 et une configuration fermée du passage d'accès 27B, visible sur la figure 2, empêchant l'accès à l'unité de décontamination 24.

La paroi d'obturation 29 est mobile en translation ou/et en rotation de manière relative par rapport au passage d'accès 27B entre la configuration ouverte et la configuration fermée.

Dans l'exemple illustré sur la figure 1 et 2, la paroi d'obturation 29 est un panneau monté mobile, de préférence en translation, sur le boitier 27.

Comme illustré sur la figure 2, l'unité de décontamination 24 comporte un support 32, un organe de décontamination 34, et un mécanisme 36 de déplacement relatif entre le support 32 et l'organe de décontamination 34, entre une position de repos destinée au chargement de l'objet creux 12 dans le support 32 (visible sur la figure 1) et une position de décontamination, dans laquelle l'organe de décontamination 34 est inséré dans la cavité 14 de l'objet creux 12 (visible sur la figure 2).

Le support 32 est propre à positionner et maintenir l'objet creux 12 dans l'appareil 10 en évitant l'introduction d'un objet creux 12 inadapté à l'enceinte 26 et à l'unité de décontamination 24, en particulier à l'organe de décontamination 34.

Dans cet exemple, le support 32 comporte une coque 40 définissant au moins une empreinte 42 de forme complémentaire à au moins une partie de l'objet creux 12, et avantageusement un cavalier 44 de réception du col 22.

Le support 32 comporte ici avantageusement un volet 46 mobile de verrouillage du positionnement de l'objet creux 12 dans le support 32, monté mobile sur la coqué 40.

La coque 40 est avantageusement une pièce de forme, notamment fabriquée en métal, en plastique ou en résine, par exemple grâce à une technologie d'impression 3D.

Ainsi, le support 32 est configuré pour assurer une coaxialité entre la cavité interne 14 de l'objet creux 12, en particulier l'axe A-A' de l'ouverture d'accès 16 de l'objet creux 12, et l'axe B-B' de déplacement relatif de l'organe de décontamination 34 par rapport au support 32.

Comme on le verra plus bas, le support 32 est également configuré pour assurer une fermeture sans difficulté de l'enceinte 26, et à permettre un déplacement relatif entre l'organe de décontamination 34, le support 32, et l'objet creux 12, lorsque l'objet creux 12 est reçu dans le support 32.

L'empreinte 42 est ménagée dans la coque 40. Elle présente une forme complémentaire à au moins une région du récipient 20.

Dans une variante, le support 32 définit une pluralité d'empreintes 42 adaptées à des objets creux 12 de formes et/ou de dimensions différentes.

Le cavalier 44 est destiné à recevoir le col 22. Il présente également une forme complémentaire à au moins une surface extérieure du col 22.

L'empreinte 42 et le cavalier 44 définissent la position le long de l'axe de déplacement B-B' et l'orientation de l'objet creux 12 lorsque l'objet creux 12 est reçu dans le support 32. Ils forment une région de guidage ou/et de réception de l'objet creux 12 dans le support 32.

Éventuellement, l'empreinte 42 ou/et le cavalier 44 présentent au moins un relief d'indexation angulaire (non représenté), permettant d'orienter l'objet creux 12 autour de son axe longitudinal A-A' selon une orientation prédéfinie.

Le volet 46 est monté mobile sur la coque 40 entre une position de mise en place de l'objet creux 12 dans le support 32, visible sur la figure 1, et une position de maintien en place de l'objet creux 12 dans le support 32, visible sur la figure 2.

Dans cet exemple, le volet 46 est articulé sur la coque 40. Il est pivotable par rapport à la coque 40 entre la position de mise en place et la position de maintien. En variante, non représentée, le volet 46 est déplaçable en translation par rapport à la coque 40.

Le support 32 comporte de préférence un élément de verrouillage (non représenté) du volet 46 dans la position de maintien. Cet élément de verrouillage est par exemple actionné manuellement par un utilisateur de l'appareil 10, ou par un actionneur piloté par l'unité de commande 30, notamment un actionneur électromécanique.

Dans une variante non représentée, le support 32 est dépourvu de volet 46. L'empreinte 42 présente alors une forme adaptée et suffisante pour prévenir tout déplacement de l'objet 12 placé dans le support 32, notamment lors d'un déplacement relatif entre le support 32 et l'organe de décontamination 34.

L'organe de décontamination 34 est choisi en fonction de la méthode de décontamination mise en oeuvre, qui peut être toute méthode connue de l'homme de l'art.

La forme et les dimensions de l'organe de décontamination 34 sont choisies pour autoriser son insertion dans la cavité interne 14, à travers l'ouverture 16, en fonction des dimensions et/ou de la forme de la cavité interne 14 et si nécessaire de l'ouverture 16.

De préférence, l'organe de décontamination 34 présente une longueur choisie pour rester à l'écart du fond de la cavité interne 14.

Dans un exemple, l'organe de décontamination 34 est propre à émettre de la vapeur dans la cavité interne 14, en particulier de la vapeur sèche, à diffuser de l'ozone dans la cavité interne 14 (voir par exemple EP0436042A1, US20060032189A1) ou/et à pulvériser une solution désinfectante et de rinçage.

En variante ou en complément, l'organe de décontamination 34 est propre à émettre une lumière, par exemple une lumière DEL, une lumière UV (voir par exemple EP2816002), ou/et à émettre des radiations électromagnétiques (voir par exemple US2007258851, US6085492).

De préférence, l'organe de décontamination 34 repose sur une technique de décontamination anhydre.

Avantageusement, l'organe de décontamination 34 repose sur une technique de décontamination de type lumière DEL ou UV, avantageusement sous forme de lumière pulsée, et mieux de lumière UV. Une lumière UV est de préférence une lumière ayant un pic d'émission de longueur d'onde inférieure à 380 nm, avantageusement comprise entre 10 nm et 380 nm.

L'organe de décontamination 34 comporte alors une source lumineuse 50, notamment une lampe, de préférence en quartz, de type xénon moyenne pression fonctionnant, optionnellement en mode pulsé, et optionnellement pourvu d'un réflecteur radiofréquence pour décontaminer également le col 22 de l'objet creux 12. Il comporte en outre une alimentation 52 de la source lumineuse 50, propre à être pilotée par l'unité de commande 30.

La source lumineuse 50 est destinée à être positionnée dans la cavité 14, de telle sorte qu'une partie du corps rayonnant de cette source lumineuse 50 soit en saillie hors de la cavité de l'objet creux 12.

De préférence, la source lumineuse 50 présente une forme allongée selon un axe B-B' de déplacement de l'organe de décontamination 34 par rapport au support 32, en particulier une forme cylindrique ou oblongue.

La source lumineuse 50 peut aussi contribuer à la désinfection par un effet thermique ou/et un effet corps noir.

Un organe de décontamination 34 est notamment tel que décrit dans WO2010012915.

L'organe de décontamination 34 est déplaçable relativement au support 32 via le mécanisme de déplacement 36, entre la position de repos destinée au positionnement de l'objet creux 12 dans le support 32 et la position de décontamination, dans laquelle au moins une partie de l'organe de décontamination 34 est destiné à être inséré dans la cavité 14 de l'objet creux 12.

Ce déplacement est avantageusement une translation le long de l'axe B-B' de l'organe de décontamination 34.

Dans l'exemple représenté sur les figures 1 et 2, le support 32 est mobile dans l'enceinte 26 et l'organe de décontamination 34 est statique par rapport à l'enceinte 26. Le mécanisme de déplacement 36 est propre à engendrer un déplacement du support 32 par rapport à l'organe de décontamination 34.

Dans une variante, non représentée, le support 32 est statique par rapport à l'enceinte 26 et l'organe de décontamination 34 est mobile dans l'enceinte 26. Le mécanisme de déplacement 36 est propre à engendrer un déplacement de l'organe de décontamination 34 par rapport au support 36.

Dans encore une autre variante, non représentée, le support 32 et l'organe de décontamination 34 sont mobiles dans l'enceinte 26. Le mécanisme de déplacement 36 est propre à engendrer un déplacement simultané de l'organe de décontamination 34 et du support 36 pour qu'ils se rapprochent ou s'éloignent l'un de l'autre.

Dans cet exemple, le mécanisme de déplacement 36 comporte un actionneur 60, par exemple électromécanique, destiné à être piloté par l'unité de commande 30 ou par un bouton de commande actionné par l'utilisateur, pour provoquer le déplacement relatif entre le support 36 et l'organe de décontamination 34.

Le mécanisme de déplacement 36 comporte avantageusement, un organe de guidage 62, sur lequel le support 32 ou/et l'organe de décontamination 34 est destiné à coulisser lors du déplacement entre la position de repos et la position de décontamination. Ceci évite toute mauvaise manipulation du mécanisme de déplacement 36.

En variante, le mécanisme de déplacement 36 comporte un organe de déplacement manuel, à la place de l'actionneur 60, par exemple une poignée ou une manette. Le déplacement relatif entre le support 32 et l'organe de décontamination 34 est alors réalisé manuellement par l'utilisateur de l'appareil 10, ce qui simplifie le mécanisme de déplacement 36 et assure sa robustesse.

Dans la position de repos, telle qu'illustrée par la figure 1, le support 32 est éloigné relativement de l'organe de décontamination 34 le long de l'axe de déplacement B-B'. La paroi d'obturation 29 occupe de préférence sa configuration ouverte.

Le support 32 est disposé en regard du passage d'accès 27B, l'organe de décontamination 34 étant de préférence disposé à l'écart du passage d'accès 27B en étant masqué par une cloison de l'enceinte 26.

Cette position autorise la mise en place de l'objet creux 12 dans le support 32, en limitant le risque de contact avec l'organe de décontamination 34.

Dans une variante, dans la position de repos, le support 32 fait saillie hors de l'enceinte 26 à travers le passage d'accès 27B.

Dans la position de décontamination, l'organe de décontamination 34 et le support se sont rapprochés l'un de l'autre le long de l'axe B-B', pour que l'organe de décontamination 34 se place en regard du support 32, et s'insère dans la cavité interne 14 de l'objet creux 12, de préférence sans contact avec l'objet creux 12, au moins avec le col 22 et/ou le fond de l'objet creux 12.

La paroi d'obturation 29 occupe alors sa configuration fermée, empêchant l'accès au volume intérieur 27A du boîtier 27 à travers le passage d'accès 27B et obturant de manière opaque le boitier 27.

Avantageusement, le déplacement relatif de l'organe de décontamination 34 par rapport au passage d'accès 27B, de la position de repos à la position de décontamination, provoque le déplacement relatif de la paroi d'obturation 29 par rapport au support 32, de la configuration ouverte à la configuration fermée. Avantageusement, le déplacement relatif de la paroi d'obturation 29 par rapport au passage d'accès 27B, de la configuration ouverte à la configuration fermée, provoque le déplacement relatif de l'organe de décontamination 34 par rapport au support 32, de la position de repos à la position de décontamination.

Le mécanisme de déplacement 36 comporte de préférence un ensemble de couplage de l'organe de décontamination 34 et/ou du support 32 avec la paroi d'obturation 29 pour assujettir le déplacement relatif de la paroi d'obturation 29 par rapport au passage d'accès 27B au déplacement relatif entre l'organe de décontamination 34 et le support 32.

Lorsque le support 32 est mobile, l'organe de décontamination 34 étant statique, l'ensemble de couplage assujettit le déplacement relatif de la paroi d'obturation 29 par rapport au passage 27B au déplacement du support 32.

Lorsque le support 32 est statique, l'organe de décontamination 34 étant mobile, l'ensemble de couplage assujettit le déplacement relatif de la paroi d'obturation 29 par rapport au passage d'accès 27B au déplacement de l'organe de décontamination 34.

Dans une variante, la paroi d'obturation 29 est maintenue en permanence dans la configuration fermée, y compris dans la position de repos, par exemple en étant sollicitée par un ressort. Dans ce cas, l'insertion de l'objet creux 12 dans le support 32 nécessite que l'utilisateur ou l'unité de commande 30 passe préalablement la paroi d'obturation 29 dans la configuration ouverte, le support 32 étant dans la position de repos.

Plus généralement, tous les déplacements sont engendrés manuellement par l'utilisateur ou en variante, par l'unité de commande 30.

Le système de capteurs 28 comporte un premier capteur 70 de détection de la mise en place de l'objet creux 12 dans le support 32, un deuxième capteur 72 de détection de la configuration fermée de la paroi d'obturation 29, et/ou un troisième capteur 74 de détection de la position de décontamination de l'organe de décontamination 34. Il comporte avantageusement un quatrième capteur 76 de détection de la position du volet 46.

Les capteurs 70 à 74 sont raccordés à l'unité de commande 30.

Le premier capteur 70 est propre à identifier la présence d'un objet creux 12 placé dans le support 32, et ainsi de permettre à l'unité de commande 30 d'autoriser ou non l'activation du procédé de décontamination décrit plus bas.

Avantageusement, le premier capteur 70 est en outre capable d'identifier la forme et/ou le poids de l'objet creux 12 positionné sur le support 32, notamment de manière à ajuster le déplacement relatif de l'organe de décontamination 34 par rapport au support 32 et/ou la séquence de décontamination.

Il est propre de préférence à détecter un mauvais positionnement de l'objet creux 12 relativement à l'organe de décontamination 34 pour permettre à l'unité de commande 30 d'empêcher le déplacement relatif entre le support 32 et l'organe de décontamination 34 et toute séquence de décontamination.

Avantageusement, le capteur 70 peut être couplé à au moins un indicateur lumineux visible de l'opérateur ou du consommateur renseignant sur la présence d'un objet creux 12 adapté au et correctement placé dans le support 32. En variante, le capteur 70 peut être couplé à au moins deux indicateurs lumineux, de préférence de couleurs différentes, visibles de l'opérateur ou du consommateur, le premier indicateur renseignant sur l'absence d'objet creux 12 dans le support 32 ou sur la présence d'un objet creux 12 non adapté au ou non correctement placé dans le support 32 et le deuxième indicateur lumineux renseignant sur la présence d'un objet creux 12 adapté au et correctement placé dans le support 32.

Le deuxième capteur 72 est de préférence apte à détecter la configuration fermée et la configuration ouverte de la paroi d'obturation 29, pour permettre à l'unité de commande 30 respectivement d'autoriser ou d'empêcher l'activation de l'organe de décontamination 34 ou/et le déplacement relatif du support 32 par rapport à l'organe de décontamination 34.

Le troisième capteur 74 est apte avantageusement à détecter au moins la position de repos et la position de décontamination.

Le quatrième capteur 76 est propre à détecter la position de maintien du volet 46 pour permettre avantageusement à l'unité de commande 30 de verrouiller en position le volet 46 dans cette position.

L'unité de commande 30 comporte au moins un processeur et une mémoire contenant des modules logiciels propres à être exécutés par le processeur. Elle comporte une interface propre à communiquer avec le ou chaque capteur 70 à 76 du système de capteur 28 et notamment propre à relever la présence de l'objet creux 12 sur le support 32, et avantageusement son format et/ou son poids et/ou son positionnement adéquat.

Elle comporte un module d'activation sélective du déplacement relatif entre le support 32 et l'organe de décontamination 34 vers la position de décontamination, et un module d'activation de l'organe de décontamination 34 pour engendrer une décontamination, sur la base des données acquises par les capteurs 70 à 76.

Ainsi, l'unité de commande 30 est propre à activer l'organe de décontamination 34. Par « activer l'organe de décontamination », on entend que l'unité de commande 30 est propre au moins à rendre actif l'organe de décontamination 34 dans la mise en oeuvre d'un traitement de décontamination, par exemple en allumant la source lumineuse 50, en démarrant la distribution de vapeur, de gaz ou de solution désinfectante et de rinçage par l'organe de décontamination 34.

Eventuellement, comme on le verra plus bas, l'activation de l'organe de décontamination 34 s'accompagne ou est précédé de la commande du déplacement relatif de l'organe de décontamination 34 par rapport au support 32 depuis la position de repos vers la position de décontamination.

L'unité de commande 30 comprend avantageusement au moins un module de détection de problèmes au niveau de la paroi d'obturation 29, du support 32, du mécanisme de déplacement 36 ou/et de l'organe de décontamination 34 ou/et optionnellement du volet 46.

Avantageusement, en l'absence d'objet creux 12 porté par le support 32, l'unité de commande 30 est configurée pour piloter un retour automatique dans une configuration d'attente, dans laquelle la paroi d'obturation 29 est dans la configuration fermée. Dans ce cas, l'organe de décontamination 34 occupe éventuellement la position de décontamination par rapport au support 32, sans être activé.

De préférence, notamment dans l'appareil 10 représenté sur les figures 1 et 2, le retour dans la configuration d'attente est piloté automatiquement après une temporisation d'une durée comprise entre 5 secondes et 30 secondes, en particulier entre 10 secondes et 20 secondes, et mieux entre 10 secondes et 15 secondes.

Selon un premier mode de réalisation, l'unité de commande 30 comprend au moins un bouton physique ou logiciel apte à être pressé par l'utilisateur pour initier le procédé de décontamination décrit plus loin.

Selon une variante, l'unité de commande 30 comprend au moins deux boutons physiques ou logiciels, où :
- le premier bouton est apte à assurer le déplacement relatif de la paroi d'obturation 29 par rapport au passage d'accès 27B entre la configuration ouverte et la configuration fermée, voire le déplacement relatif de l'organe de décontamination 34 par rapport au support entre la position de repos et la position de décontamination, et
- le deuxième bouton est apte à être pressé par un utilisateur pour assurer au moins l'activation l'organe de décontamination 34.

De préférence, le boitier peut comprendre en outre au moins un bouton d'arrêt d'urgence.

Un procédé de décontamination d'un objet creux 12, mis en oeuvre à l'aide de l'appareil de décontamination 10 selon l'invention, va maintenant être décrit.

Initialement, l'appareil de décontamination 10 est fourni dans la position de repos de l'organe de décontamination 30 par rapport au support 32 et dans la configuration ouverte de la paroi d'obturation 29 par rapport au passage d'accès 27B.

Selon une variante, la paroi d'obturation 29 est préalablement passée d'une configuration fermée à une configuration ouverte relativement au passage d'accès 27B et l'organe de décontamination 30 est passé d'une position de décontamination à une position de repos relativement au support 32.

Un objet creux 12 présentant une cavité 14 est alors introduit dans le support 32 par l'utilisateur. Lorsqu'il est présent, le volet 46 est initialement placé dans la position de mise en place, puis est manoeuvré dans la position de maintien après que l'objet creux a été disposé dans le support 32. Le volet 46 est de préférence verrouillé dans la position de maintien.

Une séquence de décontamination est alors mise en oeuvre.

La paroi d'obturation 29 passe dans la configuration fermée, et le mécanisme de déplacement 36 rapproche l'organe de décontamination 34 du support 32 pour passer de la position de repos à la position de décontamination dans laquelle l'organe de décontamination 34 est inséré dans la cavité 14 de l'objet creux 12.

Lorsque la paroi d'obturation 29 est dans la configuration fermée, au moins le support 32, l'objet creux 12 et l'organe de décontamination 34 est totalement contenu dans le volume intérieur 27A de l'enceinte 26, fermé par la paroi d'obturation 29.

Optionnellement, le passage de la paroi d'obturation 29 de la configuration ouverte à la configuration fermée et le rapprochement de l'organe de décontamination 34 du support 32 pour passer de la position de repos à la position de décontamination sont simultanés.

Puis, l'organe de décontamination 34 est activé par l'unité de commande 30, sur la base des mesures reçues d'au moins un capteur 70 à 74 du système de capteurs 28.

Optionnellement, l'activation de l'organe de décontamination 34 est initiée lors du passage de la position de repos à la position de décontamination, mais uniquement après que la configuration fermée ait été atteinte.

Dans un mode de réalisation, l'organe de décontamination 34 est activé une seule fois, soit sur un temps très court (sous forme d'un flash) ou de manière continue.

Alternativement, l'organe de décontamination 34 est activé au moins deux fois, de préférence au moins trois fois, etc..., de préférence sous forme de flashs successifs. Ceci évite à l'organe de décontamination 34 de chauffer, et donc de s'abimer, et est moins gourmand en énergie. La décontamination est plus efficace, et diminue le risque d'altérer la sérigraphie pouvant être présente sur l'objet creux 12.

Avantageusement, l'organe de décontamination 34 est activé au cours de son introduction dans la cavité 14, de manière à raccourcir la durée d'une séquence de décontamination et donc la durée du procédé de décontamination et à améliorer l'efficacité de la décontamination de l'objet creux 12.

Puis, l'organe de décontamination 34 est désactivé. Le mécanisme de déplacement 36 éloigne relativement l'organe de décontamination 34 du support 32 pour passer de la position de décontamination à la position de repos. L'organe de décontamination 34 sort de la cavité 14.

La désactivation de l'organe de décontamination 34 peut intervenir lors du déplacement de la position de décontamination à la position de repos.

La paroi d'obturation 29 se déplace relativement au passage d'accès 27B pour passer de la configuration fermée à la configuration ouverte.

Dans un mode de réalisation, non représenté, lorsque le support 32 est mobile par rapport à l'enceinte 26, dans la position de repos, le support 32 fait avantageusement au moins partiellement saillie hors de l'enceinte 26.

Optionnellement, le passage de la position de décontamination à la position de repos est simultané au passage de la configuration fermée à la configuration ouverte, mais intervient uniquement après la désactivation de l'organe de décontamination 34.

Ensuite, lorsque le support comprend un volet 46, celui-ci est déverrouillé et passé dans sa position de mise en place.

L'objet creux 12 décontaminé est alors extrait hors du support 32 est récupéré par l'utilisateur, en vue de recevoir une dose de produit distribué par la machine 18.

Eventuellement, l'unité de commande 30 pilote un retour automatique dans une configuration d'attente, dans laquelle la paroi d'obturation 29 est dans la configuration fermée. Eventuellement, l'organe de décontamination 34 occupe la position de décontamination par rapport au support 32, sans être activé.

Le procédé peut alors être mis en oeuvre pour un nouvel objet creux 12, en répétant les étapes ci-dessus.

Grâce à l'appareil 10 et au procédé selon l'invention il est possible d'assurer de manière simple et sécurisée, par exemple en magasin ou sur site de démonstration (notamment un salon), une décontamination satisfaisante d'un objet creux 12 présentant une cavité 14 (notamment un emballage) pour un remplissage subséquent.

En outre, toute interaction entre l'objet creux 12 et l'organe de décontamination 34 est limitée, voire empêchée, ce qui évite ou minimise le risque de contamination et/ou de casse de l'organe de décontamination 34.

L'appareil 10 selon l'invention est donc particulièrement adapté à la mise en oeuvre ponctuelle d'une décontamination d'un objet creux 12, contrairement à une installation industrielle de décontamination fonctionnant en continu.

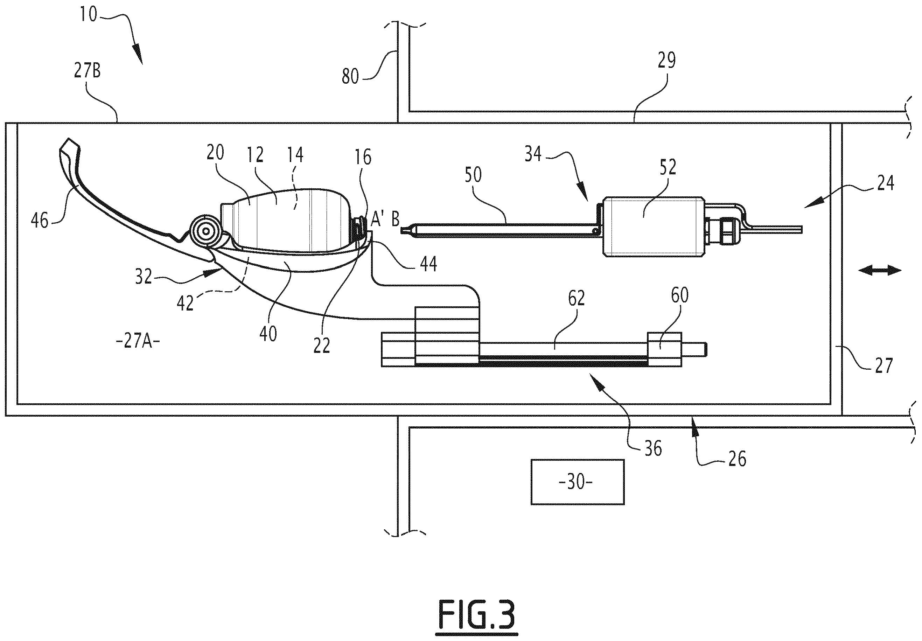

L'appareil de décontamination 10 illustré sur la figure 3 diffère de celui illustré sur la figure 1 en ce que le boîtier 27 est un tiroir déplaçable par rapport à un bâti 80 qui contient la paroi d'obturation 29. La paroi d'obturation 29 est avantageusement statique par rapport au bâti 80.

Avantageusement, le bâti 80 est celui d'une machine 18 de distribution de produit, telle qu'illustrée sur la figure 9.

Lors de l'extraction du tiroir hors du bâti 80, le passage d'accès 27B passe en configuration ouverte par rapport à la paroi d'obturation 29. Il repasse en configuration fermée lorsque le tiroir s'insère dans le bâti 80.

Dans l'exemple représenté sur la figure 3, le support 32 et l'organe de décontamination 34 se déplacent conjointement avec le tiroir, lors de l'extraction du tiroir hors du bâti 80. En variante, seul le support 32 ou seul l'organe de décontamination 34 se déplace conjointement avec le tiroir, l'ouverture du tiroir provoquant le passage de l'organe de décontamination 34 dans la position de repos par rapport au support 32, la fermeture du tiroir provoquant le passage de l'organe de décontamination 34 dans la position de décontamination par rapport au support 32.

Le fonctionnement de l'appareil de décontamination 10 illustré sur la figure 3 et par ailleurs analogue à celui illustré sur la figure 1.

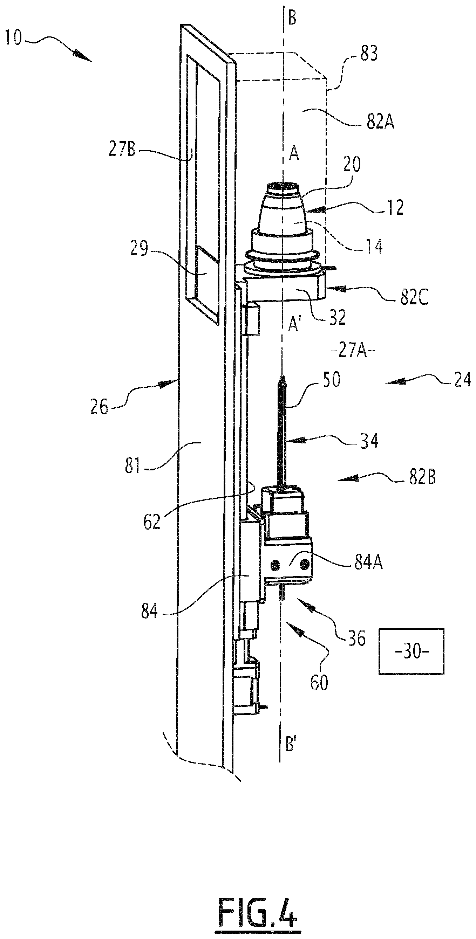

Dans la variante illustrée par les figures 4 à 6, l'appareil 10 comporte une enceinte 26 présentant une cloison 81 avantageusement verticale définissant le passage d'accès 27B débouchant dans le volume intérieur 27A.

Le volume intérieur 27B de l'enceinte 26 comprend une région 82A d'accès au support 32 débouchant vers l'extérieur de l'appareil 10 via le passage d'accès 27B, et une région technique 82B, avantageusement séparée de la région d'accès 82A par au moins une cloison 82C interne au volume intérieur, ici une cloison horizontale.

La région d'accès 82A est fermée vers l'intérieur par un capot 83, disposé à l'opposé du passage d'accès 27B. Ainsi, la région d'accès 82A et le passage d'accès 27B définissent une alcôve ménagée dans l'enceinte 26.

Dans cet exemple, la région d'accès 82A est située au-dessus de la région technique 82B.

Le support 32 est contenu dans la région d'accès 82A. Il est ici statique, notamment par rapport à la cloison 81. Lorsque l'objet creux 12 est monté sur le support 32, il est totalement contenu dans la région d'accès 82A.

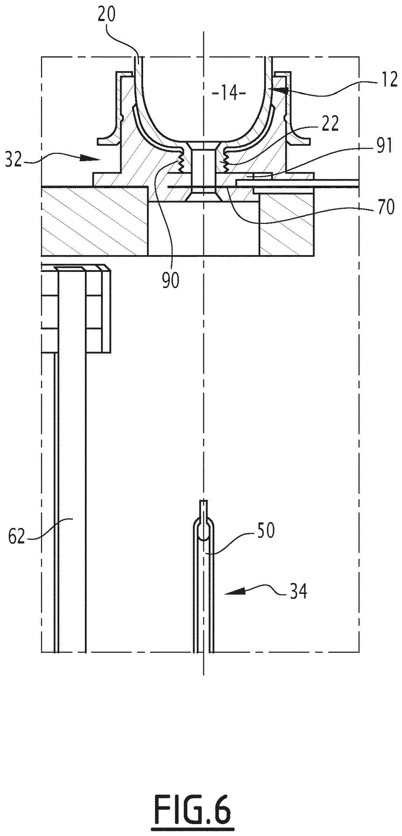

En référence à la figure 6, le support 32 comporte un manchon 90 de positionnement du col 22 de l'objet creux 12, complémentaire d'au moins l'extrémité du col 22 de l'objet creux 12, par exemple d'un goulot de l'objet creux 12. Le manchon 90 forme une région de guidage et de positionnement de l'objet creux 12.

De préférence, le col 22 se visse sur le manchon 90. Le manchon 90 définit alors un filetage interne complémentaire d'un filetage externe sur le col 22 de l'objet creux 12.

Lors de l'insertion de l'objet creux 12 dans le support 32, l'objet creux 12 est positionné dans le manchon 90 jusqu'à ce que le buvant du col 22 atteigne une butée 91 qui actionne le premier capteur 70 de présence, ici inséré sous la butée 91.

Le manchon 90 guide linéairement l'objet creux 20 et garantit ainsi une introduction fiable de l'organe de décontamination 34 dans la cavité 14, au maximum de sa course. Un guidage le plus satisfaisant possible et donc une coaxialité avec l'organe de décontamination 34 la plus satisfaisante possible est ainsi assurée.

L'organe de décontamination 34 est monté mobile par rapport au support 32, en particulier, mobile en translation le long d'un axe vertical B-B', entre la position de repos et la position de décontamination.

Dans la position de repos, l'organe de décontamination 34 est ici totalement contenu dans la région technique 82B, à l'écart de la région d'accès 82A et du passage d'accès 27B.

Dans la position de décontamination, l'organe de décontamination 34 est au moins partiellement inséré dans la région d'accès 82A, afin de s'introduire dans la cavité interne 14 de l'objet creux 12 monté sur le support 32. En particulier, l'organe de décontamination 34 fait saillie à travers le manchon 90 pour atteindre la cavité interne 14.

La paroi d'obturation 29 est ici un panneau monté mobile par rapport au passage d'accès 27B entre la configuration ouverte et la configuration fermée.

Le déplacement de la paroi d'obturation 29 est ici conjoint avec celui de l'organe de décontamination 34.

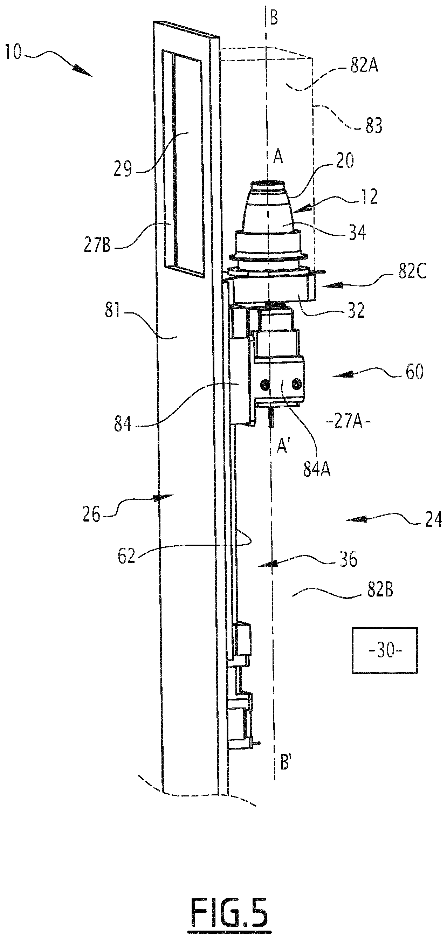

Ainsi, de préférence, la paroi d'obturation 29 est mobile en translation parallèlement à l'axe de déplacement B-B' (ici vertical), entre la configuration ouverte, visible sur la figure 4 et la configuration fermée, visible sur la figure 5.

Dans la configuration ouverte, la paroi d'obturation 29 est au moins partiellement escamotée (ici vers le bas), pour libérer au moins partiellement le passage d'accès 27B et autoriser un utilisateur à charger ou décharger un objet creux 12 dans le support 32. L'alcôve est alors accessible.

Dans la configuration fermée, la paroi d'obturation 29 obture tout le passage d'accès 27B, pour empêcher un utilisateur de charger ou décharger un objet creux 12 dans le support 32 et avantageusement pour le protéger de toute lumière ou de toute projection de produit à partir de l'organe de décontamination 34. L'alcôve est alors inaccessible.

En outre, le support 32 est avantageusement disposé verticalement au-dessus de l'organe de décontamination 34, l'objet creux 12 étant alors placé avec la cavité interne 14 débouchant vers le bas. Ceci évite qu'en cas de rupture de l'organe de décontamination 34 par exemple due à un choc, des résidus s'introduisent dans la cavité 14.

Pour assurer un déplacement précis de l'organe de décontamination 34 par rapport au support 32, et maintenir l'alignement de l'organe de décontamination 34 par rapport à l'objet creux 12 monté dans le support 32, l'actionneur 60 comporte un chariot mobile 84 monté coulissant sur au moins une tige de guidage 62, pour porter l'organe de décontamination 34. Le chariot mobile 84 comporte deux équerres latérales 84A, disposées parallèles l'une à l'autre de part et d'autre de l'axe de déplacement B-B'. L'organe de décontamination 34, en particulier le générateur 52 est monté enserré entre les équerres 84A.

L'appareil 10 illustré par les figures 4 à 6 est avantageusement un dispositif autonome, par exemple une borne, la cloison 81 formant une surface extérieure de la borne. En variante, l'appareil 10 illustré par les figures 4 à 6 est intégré au sein d'une machine 18 de distribution de produit en vrac dans l'objet creux 12, la cloison 81 formant de préférence une paroi extérieure de la machine 18.

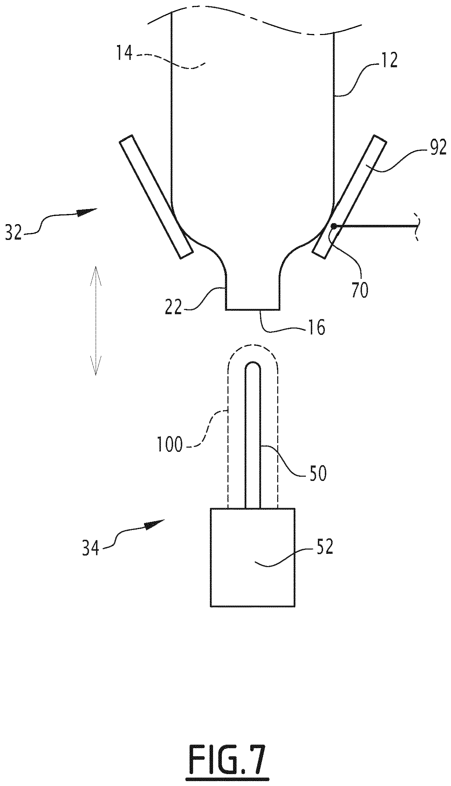

Dans une variante illustrée par la figure 7, le support 32 comporte une région de guidage et de positionnement adaptée pour positionner et maintenir l'objet creux 12 par simple gravité, et notamment par un guidage au moins du col 22 par glissement sur la région de guidage et de positionnement.

La région de guidage et de positionnement comporte une surface de révolution 92 convergente vers le bas, par exemple une surface tronconique.

Dans cette variante, l'unité de décontamination 24 est avantageusement positionnée selon un axe vertical. Le support 32 est configuré pour recevoir l'objet 12 tête en bas. Il est placé au-dessus de l'organe de décontamination 34.

Cette variante est avantageuse en ce qu'elle évite de forcer la coaxialité entre l'objet creux 12 et l'organe de décontamination 34, en autorisant un jeu de déplacement entre l'objet creux 12 et l'organe de décontamination 34, même en présence d'espacements très faibles entre l'organe de décontamination 34 et le col 22 de l'objet creux 12.

Dans une variante (représentée en pointillés sur la figure 7), l'unité de décontamination 24 comprend avantageusement une enveloppe extérieure 100 coaxiale avec l'organe de décontamination 34. L'enveloppe 100 présente une extrémité ouverte en forme d'entonnoir au niveau de l'extrémité libre de l'organe de décontamination 34 destinée à entrer dans la cavité 14 de l'objet creux 12. L'extrémité de l'enveloppe 100 est configurée pour pénétrer au moins une partie du col 22 de l'objet creux 12 pour permettre le passage de l'organe de décontamination 34. L'enveloppe 100 assure ainsi un double guidage de l'organe de décontamination 34 dans l'objet creux 12 et prévient, voire empêche efficacement tout risque de contact, voire de collision, entre l'organe de décontamination 34 et le col 22 de l'objet creux 12.

## Revendications

1. Appareil (10) de décontamination d'un objet creux (12) définissant une cavité interne (14), comportant :
- une unité de décontamination (24), l'unité de décontamination (24) comportant un support (32) destiné à recevoir et à positionner l'objet creux (12), un organe de décontamination (34) destiné à être introduit dans la cavité interne (14), et un mécanisme de déplacement (36) relatif de l'organe de décontamination (34) par rapport au support (32) propre à déplacer l'organe de décontamination (34) et le support (32) relativement l'un par rapport à l'autre entre une position de repos destinée au positionnement de l'objet creux (12) dans le support (32) et une position de décontamination, dans laquelle l'organe de décontamination (34) est destiné à être inséré dans la cavité interne (14) de l'objet creux (12) reçu dans le support (32),
- une unité de commande (30) propre à activer l'organe de décontamination (34) dans la position de décontamination,
**caractérisé par** :
- une enceinte (26) de protection définissant un volume intérieur (27A) contenant au moins une partie de l'organe de décontamination (24) et le support (32) au moins dans la position de décontamination, l'enceinte (26) définissant un passage d'accès (27B) au volume intérieur (27A) et au moins une paroi (29) d'obturation du passage d'accès (27B), la paroi d'obturation (29) et le passage d'accès (27B) étant mobiles l'un par rapport à l'autre entre une configuration ouverte du passage d'accès (27B) que la paroi d'obturation (29) est propre à occuper dans la position de repos de l'organe de décontamination (34) et une configuration fermée du passage d'accès (27B) que la paroi d'obturation (29) occupe dans la position de décontamination de l'organe de décontamination (34).

2. Appareil (10) selon la revendication 1, dans lequel le déplacement relatif de l'organe de décontamination (34) par rapport au passage d'accès (27B) de la position de repos à la position de décontamination provoque le déplacement relatif de la paroi d'obturation (29) par rapport au support (32) de la configuration ouverte à la configuration fermée,
ou/et dans lequel le déplacement relatif de la paroi d'obturation (29) par rapport au passage d'accès (27B) de la configuration ouverte à la configuration fermée provoque le déplacement relatif de l'organe de décontamination (34) par rapport au support (32) de la position de repos à la position de décontamination,
le déplacement relatif de l'organe de décontamination (34) par rapport au support (32) et le déplacement relatif de la paroi d'obturation (29) par rapport au passage d'accès (27B) étant de préférence assujettis l'un à l'autre.

3. Appareil (10) selon la revendication 1 ou 2 dans lequel dans la position de repos, la paroi d'obturation (29) est déplaçable relativement au passage d'accès (27B) entre la configuration ouverte et la configuration fermée.

4. Appareil (10) selon la revendication 3, dans lequel la paroi d'obturation (29) est maintenue au repos dans la configuration fermée.

5. Appareil (10) selon l'une quelconque des revendications précédentes, dans lequel l'unité de commande (30) est propre à piloter le déplacement relatif de l'organe de décontamination (34) par rapport au support (32) entre la position de repos et la position de décontamination ou/et est propre à piloter le déplacement relatif de la paroi d'obturation (29) par rapport au passage d'accès (27B) de la configuration ouverte à la configuration fermée.

6. Appareil (10) selon l'une quelconque des revendications précédentes comportant un premier capteur (70) de détection de la mise en place de l'objet creux (12) dans le support (32) et/ou un deuxième capteur (72) de détection de la configuration fermée de la paroi d'obturation (29), et/ou un troisième capteur (74) de détection de la position de décontamination, l'unité de commande (30) étant propre à activer l'organe de décontamination (34) lorsque le premier capteur (70) détecte que l'objet est mis en place dans le support (32) ou/et lorsque le deuxième capteur (72) détecte que la paroi d'obturation (29) est dans la configuration fermée, ou/et lorsque le troisième capteur (74) détecte que l'organe de décontamination (34) est dans la position de décontamination.

7. Appareil (10) selon l'une quelconque des revendications précédentes, dans lequel la paroi d'obturation (29) est un panneau déplaçable en translation ou en rotation par rapport au passage d'accès (27B) ou dans lequel l'enceinte (26) comprend un tiroir mobile définissant le passage d'accès (27B), le tiroir étant mobile par rapport à la paroi d'obturation (29).

8. Appareil (10) selon l'une quelconque des revendications précédentes, dans lequel le support (32) comporte une région de guidage ou/et de réception d'au moins une partie de l'objet creux (12), notamment d'un col (22) de l'objet creux (12), propre à fixer la position et l'alignement de l'objet creux (12) dans le support (32), la région de guidage ou/et de réception étant en particulier un manchon (90) complémentaire d'un col de l'objet creux (12), une surface (92) de guidage par gravité d'un col (22) de l'objet creux (12) ou une empreinte (42) de réception de l'objet creux (12) de forme complémentaire à l'objet creux (12), propre à fixer la position et l'alignement de l'objet creux (12) dans le support (32).

9. Appareil (10) selon la revendication 8, dans lequel le support (32) comporte un volet (46) de maintien de l'objet creux (12) dans la région de guidage ou/et de réception, déplaçable, en particulier par pivotement, entre une position de mise en place de l'objet creux (12) dans la région de guidage ou/et de réception et une position de maintien de l'objet creux (12) dans la région de guidage ou/et de réception.

10. Appareil (10) selon l'une quelconque des revendications précédentes, dans lequel l'organe de décontamination (34) comporte une source lumineuse, en particulier une source d'émission de lumière UV, continue ou pulsée.

11. Appareil (10) selon l'une quelconque des revendications précédentes, dans lequel le support (32) est disposé au-dessus de l'organe de décontamination (34) dans la position de repos.

12. Machine (18) de distribution de produit en vrac dans un objet creux (12) définissant une cavité interne (14), la machine (18) comprenant au moins un réservoir (18A) de produit, au moins une buse (18B) de distribution destinée à être placée en regard de la cavité interne (14) de l'objet creux (12), et éventuellement un ensemble (18C) de mesure de la quantité de produit introduite dans la cavité interne (14) de l'objet creux (12), la machine (18) comportant en outre un appareil (10) de décontamination selon l'une quelconque des revendications précédentes.

13. Procédé de décontamination d'un objet creux (12) présentant une cavité interne (14) comprenant les étapes suivantes :
- fourniture d'un appareil (10) de décontamination selon l'une quelconque des revendication 1 à 11 ;
- mise en oeuvre d'une séquence de décontamination comprenant les phases suivantes :
* l'organe de décontamination (34) étant dans sa position de repos, la paroi d'obturation (29) étant dans sa configuration ouverte, introduction d'un objet creux (12) présentant une cavité interne (14) dans le support (32),
* passage de la paroi d'obturation (29) dans la configuration fermée et passage de l'organe de décontamination (34) dans la position de décontamination, l'organe de décontamination (34) s'insérant dans la cavité interne (14) ;
* activation de l'organe de décontamination (34) par l'unité de commande (30) ; et, à la fin de la séquence de décontamination :
- passage de la paroi d'obturation (29) dans la configuration ouverte et passage de l'organe de décontamination (34) dans la position de repos, l'organe de décontamination (34) sortant de la cavité interne (14) ;
- retrait de l'objet creux (12) hors du support (32).

14. Procédé selon la revendication 13, dans lequel l'activation de l'organe de décontamination (34) s'effectue après le passage de la paroi d'obturation (29) dans la configuration fermée et avant que l'organe de décontamination (34) atteigne sa position de décontamination.

15. Procédé selon la revendication 13 ou 14, dans lequel, après le retrait de l'objet creux (12) hors du support (32), l'unité de commande (30) pilote le passage de la paroi d'obturation (29) dans la configuration fermée et/ou le passage de l'organe de déplacement de la position de repos à la position de décontamination, sans activer l'organe de décontamination.

16. Procédé selon la revendication 15, comportant ensuite les étapes suivantes :
- fourniture d'un nouvel objet creux (12) à décontaminer ;
- passage de l'organe de décontamination (34) dans sa position de repos, et/ou de la paroi d'obturation (29) dans sa configuration ouverte,
- mise en oeuvre d'une nouvelle séquence de décontamination comprenant les phases suivantes :
* introduction du nouvel objet creux (12) présentant une cavité interne (14) dans le support (32),
* passage de la paroi d'obturation (29) dans la configuration fermée et passage de l'organe de décontamination (34) dans sa position de décontamination, l'organe de décontamination (34) s'insérant dans la cavité interne (14) du nouvel objet creux ;
* activation de l'organe de décontamination (34) par l'unité de commande (30).

## Patentansprüche

1. Vorrichtung (10) zur Dekontamination eines hohlen Objekts (12), das einen inneren Hohlraum (14) definiert, umfassend:
- eine Dekontaminationseinheit (24), die Dekontaminationseinheit (24) umfassend eine Halterung (32) zum Aufnehmen und Positionieren des hohlen Objekts (12), ein Dekontaminationsorgan (34), das dazu bestimmt ist, in den inneren Hohlraum (14) eingeführt zu werden, und einen relativen Bewegungsmechanismus (36) des Dekontaminationsorgans (34) in Bezug auf die Halterung (32), der geeignet ist, um das Dekontaminationsorgan (34) und die Halterung (32) in Bezug aufeinander zwischen einer Ruheposition, die zum Positionieren des hohlen Objekts (12) im Halterung (32) bestimmt ist, und einer Dekontaminationsposition zu bewegen, in der das Dekontaminationsorgan (34) dazu bestimmt ist, in den inneren Hohlraum (14) des hohlen Objekts (12) eingeführt zu werden, das in der Halterung (32) aufgenommen ist,
- eine Steuereinheit (30), die geeignet ist, um das Dekontaminationsorgan (34) in der Dekontaminationsposition zu aktivieren,
**gekennzeichnet durch**:
- eine Schutzhülle (26), die ein Innenvolumen (27A) definiert, das zumindest einen Teil des Dekontaminationsorgans (24) und der Halterung (32) zumindest in der Dekontaminationsposition enthält, wobei die Hülle (26) einen Zugangskanal (27B) zu dem Innenvolumen (27A) und mindestens eine Wand (29) zum Verschließen des Zugangskanals (27B) definiert, wobei die Verschlusswand (29) und der Zugangskanal (27B) in Bezug aufeinander zwischen einer offenen Konfiguration des Zugangskanals (27B), die die Verschlusswand (29) in der Ruheposition des Dekontaminationsorgans (34) einnehmen kann, und einer geschlossenen Konfiguration des Zugangskanals (27B), die die Verschlusswand (29) in der Dekontaminationsposition des Dekontaminationsorgans (34) einnimmt, beweglich sind.

2. Vorrichtung (10) nach Anspruch 1, wobei die relative Bewegung des Dekontaminationsorgans (34) in Bezug auf den Zugangskanal (27B) von der Ruheposition zu der Dekontaminationsposition die relative Bewegung der Verschlusswand (29) in Bezug auf die Halterung (32) von der offenen Konfiguration zu der geschlossenen Konfiguration bewirkt,
oder/und wobei die relative Bewegung der Verschlusswand (29) in Bezug auf den Zugangskanal (27B) von der offenen Konfiguration in die geschlossene Konfiguration die relative Bewegung des Dekontaminationsorgans (34) in Bezug auf die Halterung (32) von der Ruheposition in die Dekontaminationsposition bewirkt,
wobei die relative Bewegung des Dekontaminationsorgans (34) in Bezug auf die Halterung (32) und die relative Bewegung der Verschlusswand (29) in Bezug auf den Zugangskanal (27B) vorzugsweise voneinander abhängig sind.

3. Vorrichtung (10) nach Anspruch 1 oder 2, wobei die Verschlusswand (29) in der Ruhestellung in Bezug auf den Zugangskanal (27B) zwischen der offenen und der geschlossenen Konfiguration verschiebbar ist.

4. Vorrichtung (10) nach Anspruch 3, wobei die Verschlusswand (29) in der geschlossenen Konfiguration in Ruhestellung gehalten wird.

5. Vorrichtung (10) nach einem der vorherigen Ansprüche, wobei die Steuereinheit (30) geeignet ist, um die relative Bewegung des Dekontaminationsorgans (34) in Bezug auf die Halterung (32) zwischen der Ruheposition und der Dekontaminationsposition zu steuern, oder/und geeignet ist, um die relative Bewegung der Verschlusswand (29) in Bezug auf den Zugangskanal (27B) von der offenen Konfiguration in die geschlossene Konfiguration zu steuern.

6. Vorrichtung (10) nach einem der vorherigen Ansprüche, umfassend einen ersten Sensor (70) zum Erfassen des Einsetzens des hohlen Objekts (12) in die Halterung (32) und/oder einen zweiten Sensor (72) zum Erfassen der geschlossenen Konfiguration der Verschlusswand (29) und/oder einen dritten Sensor (74) zum Erfassen der Dekontaminationsposition, wobei die Steuereinheit (30) geeignet ist, um das Dekontaminationsorgan (34) zu aktivieren, wenn der erste Sensor (70) erfasst, dass das Objekt in den Halter (32) eingesetzt ist, oder/und wenn der zweite Sensor (72) erfasst, dass die Verschlusswand (29) in der geschlossenen Konfiguration ist, oder/und wenn der dritte Sensor (74) erfasst, dass das Dekontaminationsorgan (34) in der Dekontaminationsposition ist.

7. Vorrichtung (10) nach einem der vorherigen Ansprüche, wobei die Verschlusswand (29) eine Platte ist, die in Bezug auf den Zugangskanal (27B) translatorisch oder rotatorisch verschiebbar ist, oder wobei die Hülle (26) ein bewegliches Schubfach umfasst, das den Zugangskanal (27B) definiert, wobei das Schubfach in Bezug auf die Verschlusswand (29) beweglich ist.

8. Vorrichtung (10) nach einem der vorherigen Ansprüche, wobei die Halterung (32) einen Führungs- oder/und Aufnahmebereich mindestens eines Teils des hohlen Objekts (12), insbesondere eines Halses (22) des hohlen Objekts (12), umfasst, der geeignet ist, die Position und die Ausrichtung des hohlen Objekts (12) in der Halterung (32) zu fixieren, wobei der Führungs- oder/und Aufnahmebereich insbesondere eine zu einem Hals des hohlen Objekts (12) komplementäre Hülse (90), eine Fläche (92) zum Führen durch Schwerkraft eines Halses (22) des hohlen Objekts (12) oder eine Vertiefung (42) zum Aufnehmen des hohlen Objekts (12) mit einer zu dem hohlen Objekt (12) komplementären Form ist, die geeignet ist, um die Position und die Ausrichtung des hohlen Objekts (12) in der Halterung (32) zu fixieren.

9. Vorrichtung (10) nach Anspruch 8, wobei die Halterung (32) eine Klappe (46) zum Halten des hohlen Objekts (12) in dem Führungs- oder/und Aufnahmebereich aufweist, die insbesondere durch Schwenken zwischen einer Position zum Einsetzen des hohlen Objekts (12) in den Führungs- oder/und Aufnahmebereich und einer Position zum Halten des hohlen Objekts (12) in dem Führungs- oder/und Aufnahmebereich verschiebbar ist.

10. Vorrichtung (10) nach einem der vorherigen Ansprüche, wobei das Dekontaminationsorgan (34) eine Lichtquelle, insbesondere eine kontinuierliche oder gepulste UV-Lichtemissionsquelle, umfasst.

11. Vorrichtung (10) nach einem der vorherigen Ansprüche, wobei die Halterung (32) in Ruhestellung über dem Dekontaminationsorgan (34) angeordnet ist.

12. Maschine (18) zum Verteilen von Schüttgut in einem hohlen Objekt (12), das einen inneren Hohlraum (14) definiert, wobei die Maschine (18) mindestens einen Speicher (18A) für das Produkt, mindestens eine Ausgabedüse (18B), die dazu bestimmt ist, gegenüber dem inneren Hohlraum (14) des hohlen Objekts (12) angeordnet zu sein, und gegebenenfalls eine Anordnung (18C) zum Messen der Menge des in den inneren Hohlraum (14) des hohlen Objekts (12) eingeführten Produkts umfasst, wobei die Maschine (18) ferner umfassend eine Dekontaminationsvorrichtung (10) nach einem der vorherigen Ansprüche.

13. Verfahren zum Dekontaminieren eines hohlen Objekts (12), das einen inneren Hohlraum (14) aufweist, umfassend die folgenden Schritte:
- Bereitstellen einer Vorrichtung (10) zur Dekontamination nach einem der Ansprüche 1 bis 11;
- Durchführen eines Dekontaminationsablaufs, umfassend die folgenden Schritte:
* während das Dekontaminationsorgan (34) in seiner Ruheposition und die Verschlusswand (29) in ihrer offenen Konfiguration ist, Einführen eines hohlen Objekts (12)das einen inneren Hohlraum (14) aufweist, in die Halterung (32),
* Überführen der Verschlusswand (29) in die geschlossene Konfiguration und Überführen des Dekontaminationsorgans (34) in die Dekontaminationsposition, wobei das Dekontaminationsorgan (34) in den inneren Hohlraum (14) eingeführt wird;
* Aktivieren des Dekontaminationsorgans (34) durch die Steuereinheit (30); und, am Ende des Dekontaminationsablaufs:
- Überführen der Verschlusswand (29) in die offene Konfiguration und Überführen des Dekontaminationsorgans (34) in die Ruheposition, wobei das Dekontaminationsorgan (34) aus dem inneren Hohlraum (14) austritt;
- Entfernen des hohlen Objekts (12) aus der Halterung (32).

14. Verfahren nach Anspruch 13, wobei die Aktivierung des Dekontaminationsorgans (34) nach Überführen der Verschlusswand (29) in die geschlossene Konfiguration passiert hat und vor Überführen des Dekontaminationsorgans (34) in seine Dekontaminationsposition erfolgt.

15. Verfahren nach Anspruch 13 oder 14, wobei die Steuereinheit (30) nach Entfernen des hohlen Objekts (12) aus der Halterung (32) das Überführen der Verschlusswand (29) in die geschlossene Konfiguration und/oder das Überführen des Bewegungsorgans von der Ruheposition in die Dekontaminationsposition steuert, ohne das Dekontaminationsorgan zu aktivieren.

16. Verfahren nach Anspruch 15, danach umfassend die folgenden Schritte:
- Bereitstellen eines neuen hohlen Objekts (12), das dekontaminiert werden soll;
- Überführen des Dekontaminationsorgans (34) in seine Ruheposition und/oder der Verschlusswand (29) in ihre offene Konfiguration,
- durchführen eines neuen Dekontaminationsablaufs, umfassend die folgenden Phasen:
* Einführen des neuen hohlen Objekts (12), das einen inneren Hohlraum (14) aufweist, in die Halterung (32),
* Überführen der Verschlusswand (29) in die geschlossene Konfiguration und Überführen des Dekontaminationsorgans (34) in seine Dekontaminationsposition, wobei das Dekontaminationsorgan (34) in den inneren Hohlraum (14) des neuen hohlen Objekts eingeführt wird;
* Aktivieren des Dekontaminationsorgans (34) durch die Steuereinheit (30).

## Claims

1. A device (10) for decontaminating a hollow object (12) defining an internal cavity (14), including:
- a decontamination unit (24), the decontamination unit (24) including a holder (32) intended to receive and position the hollow object (12), a decontamination member (34) intended to be introduced into the internal cavity (14), and a mechanism (36) for the relative displacement of the decontamination member (34) relative to the holder (32) able to displace the decontamination member (34) and the holder (32) relatively the one in relation to the other between a rest position intended for positioning the hollow object (12) in the holder (32) and a decontamination position, in which the decontamination member (34) is intended to be inserted into the internal cavity (14) of the hollow object (12) received in the holder (32)
- a control unit (30) able to activate the decontamination member (34) in the decontamination position,
**characterized by**:
- a protective enclosure (26) defining an inner space (27A) containing at least a portion of the decontamination member (24) and the holder (32) at least in the decontamination position, the enclosure (26) defining an access passage (27B) to the inner space (27A) and at least one wall (29) for obturating the access passage (27B) the closure wall (29) and the access passage (27B) being movable, the one relative to the other, between an open configuration of the access passage (27B) which the closure wall (29) is able to occupy in the rest position of the decontamination member (34) and a closed configuration of the access passage (27B) which the closure wall (29) occupies in the decontamination position of the decontamination member (34).

2. The device (10) according to claim 1, wherein the relative displacement of the decontamination member (34) relative to the access passage (27B) from the rest position to the decontamination position causes relative displacement of the closure wall (29) relative to the holder (32) from the open configuration to the closed configuration,
and/or wherein the relative displacement of the closure wall (29) relative to the access passage (27B) from the open configuration to the closed configuration causes the relative displacement of the decontamination member (34) relative to the holder (32) from the rest position to the decontamination position
the relative displacement of the decontamination member (34) relative to the holder (32) and the relative displacement of the closure wall (29) relative to the access passage (27B) preferably being coupled the one to the other.

3. The device (10) according to claim 1 or 2 wherein, in the rest position, the closure wall (29) is displaceable relative to the access passage (27B) between the open configuration and the closed configuration.

4. The device (10) according to claim 3, wherein the closure wall (29) is held at rest in the closed configuration.

5. The device (10) according to any one of the preceding claims, wherein the control unit (30) is able to control the relative displacement of the decontamination member (34) relative to the holder (32) between the rest position and the decontamination position and/or is able to control the relative displacement of the closure wall (29) relative to the access passage (27B) from the open configuration to the closed configuration.

6. The device (10) according to any of the preceding claims including a first sensor (70) for detecting the placement of the hollow object (12) in the holder (32) and/or a second sensor (72) for detecting the closed configuration of the closure wall (29), and/or a third sensor (74) for detecting the decontamination position, the control unit (30) being able to activate the decontamination member (34) when the first sensor (70) detects that the object is placed in the holder (32) and/or when the second sensor (72) detects that the closure wall (29) is in the closed configuration, and/or when the third sensor (74) detects that the decontamination member (34) is in the decontamination position.

7. The device (10) according to any one of the preceding claims, wherein the closure wall (29) is a panel able to be displaced in translation or rotation relative to the access passage (27B) or wherein the enclosure (26) comprises a movable drawer defining the access passage (27B), the drawer being movable relative to the closure wall (29).

8. The device (10) according to any one of the preceding claims, wherein the holder (32) includes a region for guiding and/or receiving at least a portion of the hollow object (12), in particular a neck (22) of the hollow object (12), able to fix the position and alignment of the hollow object (12) in the holder (32), the guiding and/or receiving region being, in particular, a sleeve (90) complementary to a neck of the hollow object (12), a surface (92) for guiding a neck (22) of the hollow object (12) by gravity or an indentation (42) for receiving the hollow object (12), the shape of which is complementary to that of the hollow object (12) and which is able to fix the position and the alignment of the hollow object (12) in the holder (32).

9. The device (10) according to claim 8, wherein the holder (32) includes a flap (46) for holding the hollow object (12) in the guiding and/or receiving region, displaceable, in particular pivotable, between a position for placing the hollow object (12) in the guiding and/or receiving region and a position for holding the hollow object (12) in the guiding and/or receiving region.

10. The device (10) according to any of the preceding claims, wherein the decontamination member (34) comprises a light source, in particular a continuous or pulsed UV light emission source.

11. The device (10) according to any one of the preceding claims, wherein the holder (32) is arranged above the decontamination member (34) in the rest position.

12. A machine (18) for dispensing bulk product into a hollow object (12) defining an internal cavity (14), the machine (18) comprising at least one product reservoir (18A), at least one dispensing nozzle (18B) intended to be placed facing the internal cavity (14) of the hollow object (12) and optionally an assembly (18C) for measuring the quantity of product introduced into the internal cavity (14) of the hollow object (12), the machine (18) further including a decontamination device (10) according to any of the preceding claims.

13. A method for decontaminating a hollow object (12) presenting an internal cavity (14) comprising the following steps:
- providing a decontamination device (10) according to any one of claims 1 to 11 ;
- implementing a decontamination sequence comprising the following steps:
* the decontamination member (34) being in its rest position and the closure wall (29) being in its open configuration, introducing a hollow object (12) presenting an internal cavity (14) into the holder (32)
* transition of the closure wall (29) to the closed configuration and transition of the decontamination member (34) to the decontamination position, the decontamination member (34) being inserted into the internal cavity (14);
* activation of the decontamination member (34) by the control unit (30); and, at the end of the decontamination sequence:
- transition of the closure wall (29) into the open configuration and transition of the decontamination member (34) into the rest position, the decontamination member (34) exiting the internal cavity (14);
- removing the hollow object (12) from the holder (32).

14. The method according to claim 13, wherein activation of the decontamination member (34) occurs after the transition of the closure wall (29) into the closed configuration and before the decontamination member (34) reaches its decontamination position.

15. The method according to claim 13 or 14, wherein, after the removal of the hollow object (12) from the holder (32), the control unit (30) controls the transition of the closure wall (29) into the closed configuration and/or the transition of the displacement member from the rest position to the decontamination position, without activating the decontamination member.

16. The method according to claim 15, further including the following steps:
- providing a new hollow object (12) to be decontaminated;
- transition of the decontamination member (34) to its rest position, and/or the closure wall (29) to its open configuration,
- implementation of a new decontamination sequence comprising the following steps:
* introducing the new hollow object (12) presenting an internal cavity (14) into the holder (32),
* transition of the closure wall (29) into the closed configuration and transition of the decontamination member (34) into its decontamination position, the decontamination member (34) being inserted into the internal cavity (14) of the new hollow object
* activation of the decontamination member (34) by the control unit (30).
